# EUROPEAN PATENT APPLICATION

(11) **EP 1 889 638 A1**
(43) Date of publication of application: **20.02.2008**
(21) Application number: 06705724.0
(22) Date of filing: 06.03.2006
(51) Int. Cl.: A61P 3/10

(54) **MEDICAMENTS AND FOOD FOR TREATMENT OR PREVENTION OF OBESITY AND/OR DIABETES CONTAINING CICER ARIETINUM EXTRACT**

(30) Priority: 07.03.2005 CN 200510024231; 04.11.2005 JP 2005321602
(71) Applicant: Jumpsun Bio-Medicine (Shanghai) Co., Ltd., Shanghai 201101 (CN)
(72) Inventor: GU,Yuanjun, Minhang District, Shanghai 201101 (CN); YUE, Xiaohua, Minhang District, Shanghai 201101 (CN); YANG, Ying, Minhang District, Shanghai 201100 (CN)
(74) Representative: Korga, Leokadia
(86) International application number: PCT/CN2006/000328
(87) International publication number: WO 2006/094450

(57) **Abstract**

This invention discloses the uses of chickpea (cicer arietinum) extract in the preparation of food for the prevention and treatment of obesity and non-insulin-dependent diabetes (NIDD). The invention provides a therapeutic agent and food containing chickpea extract for the prevention and treatment of obesity and NIDD. In this invention, the effect of chickpea extract to the high fat diet fed mice tested by the experiment proves that the chickpea extract can significantly decrease the levels of triglyceride, cholesterol and low density lipoproteins caused by high fat diet taken in a long period of time. Additionally, it is found in the experiments that the chickpea extract can improve the hyposensitivity to insulin. The preparation of the extract is also disclosed herein.

## Description

Field of the Invention

This invention relates to the use of chickpea extract as therapeutic agents and foods in the treatment and prevention of obesity and/or non-insulin-dependent diabetes (NIDD).

Description of the Related Art

Plant extracts ate the primary form in which plant-derived medicines are administered. In Europe and the United States, approximately 25% of all prescriptions contain at least one component in plant extracts form. In the United States, extracts from plants occupy more than 95% of the herbal medicine market, and crude drugs and other products account for less than 5%. In Germany, herbal products in the form of extract have attained 10% of the pharmaceutical market, accounting for nearly 30% of the OTC market. Obviously, plant extracts have a wide application and widespread market. In China, the development of extracts from Chinese traditional medicines is growing. Many research institutions and pharmaceutical companies have developed hundreds of Chinese herb extracts from astragalus, puerarin, hawthorn, etc. The active component present in standard Ginkgo extract is 50 times more potent than the raw material, and greatly elevates the therapeutic effect of the drug.

In the area of soybean research, more than 300 kinds of soybean isoflavones have been identified and used as functional foods in the United States, with nearly 2 billion dollars in annual sales. In Europe, Japan, South Korea and other countries and regions, soybean isoflavones, have become the best-sellers as main components of health food products. The kind of health-care foods containing soy isoflavones had reached more than several tens. According to statistics, at present, the annual demand for soybean isoflavones in the international market has reached 1,500 tons, but the actual annual production is only 300 tons.

Chickpea (Nuhut, Cicer arietinum L.) is named based on its appearance, which is in the form of eagle's head with beak-like protrusion near its navel. It is a rare species of wild pea genus of a leguminous plant. The plant originated in western Asia and the Near East region, and is grown in a warm temperate and relatively dry regions in the world. It is used as a first important edible leguminous plant in India. The chickpea has been cultivated in the Xinjiang province of China for over 2500 years and has been accepted as a natural Uighur traditional medicine in Xinjiang. Chickpea contains many nutrients. Its protein content is 14.9-24.6%, and its fat content is 6.4%, most of which is in the form of unsaturated fatty acids that are beneficial to human beings. For example, fat in the Kabuli type seeds contains 50.3% oleic acid and 40% linoleic acid. It is also shown that chickpea contains 3 times more amino acids as oat, and all 8 kinds of indispensable amino acids to humans are found in chickpea. Its linoleic acid content is 20% more than than of soybeans, and its trace element levels of iron, zinc, calcium, phosphor, etc. are all higher than those in other beans. All of these are playing a great role in reducing levels of plasma lipids and cholesterol, intensifying the central nervous system depression and cardio-vascular protection in middle-aged and elderly persons.

In China, as one kind of Uighur traditional medicine, chickpea has always been used in daily clinical therapies and many special secret recipes for the preparation of chickpeas have accumulated, which are especially effective in the treatment of diabetes, cardio-vascular diseases, and kidney diseases. Chickpea is also a superior nutritional food. According to investigations in Xingjiang, there are more than 600 kinds of Uighur traditional medicines in the region; 360 kinds of them are frequently used, among them about 160 kinds are originated from local resources, occupied 27% of total kinds of Uighur traditional medicines. Among the frequently used Uighur traditional medicines, about 30 kinds, including the Xingjiang checkpea, have special uses. According to ancient literature, incl. "Diet Demands", "Addendum of Chinese Materia Medica" and "Disaster-saving with Chinese Materia Medica", the chickpea possesses saliva producing and serum lipid reducing properties.

According to literature materials, it was reported continuously as early as in 1960's and 1970's that chickpea could lower serum triglyceride and cholesterol levels in experimental mice and rabbits. The inventor of this application had designed a method of free intake of foods in order to establish an animal model most close to human obesity. It was discovered that a long-term high fat diet causes remarkable increase in the body weight of mice but that chickpea is able to reduce the body weight of obese mice. Further study found that chickpea can reduce the serum LDL-C and TG levels, and elevate HDL-C levels to a great extent. It can effectively reduce the sedimentation of fat droplets in the skeletal muscle tissue and alleviate the increase of glycogen content and endovascular thickness due to high fat diet. A molecular biological study also found that the chickpea can remarkably reduce the levels of Leptin and LPL RNA, and elevate the levels of adiponectin in fat tissues of mice on long-term high-fat diet.

### SUMMARY OF THE INVENTION

One object of this invention is to solve the novel technical problem involved in the application of Chickpea extracts.

The study found that the alcoholic extract of chickpea (administered at about 0.02 g/kg/day) is more effective than crude chickpea (at about 0.5 g/kg/day). This means that the enrichment of therapeutically-effective materials in this extract is high. It was also found that the alcoholic extract is more effective than the aqueous extract, which indicated that the traditional method of water decoction is not entirely suitable for chickpea. Therefore the use of chickpea alcohol extract as raw materials of drug and food production will play a positive role in improving product quality, as well as stabilizing the curative effect, and is also beneficial for deepening the basic research of therapeutic material of chickpea and enhancement of safety of medication.

Insulin tolerance test (ITT) is an important indication of insulin sensitivity in mice by monitoring plasma glucose levels prior to and following the intra-peritoneal injection of insulin. The test can determine the sensitivity to exogenous insulin. Experiments in our study showed a significant lowering of response to exogenous insulin in high fat diet of obese mice during treatment, especially when the blood sugar was much higher than in the control group 3 hrs after medication. It was shown that insulin-resistance will be certainly improved after treatment with chickpea and its extract for 3 months, and the alcoholic extract exerts most significant effect. The chickpea extract, therefore, can be used in preparing medicines and foods to prevent and treat obesity and NIDD by increasing the sensitivity to insulin.

This invention describes the use of chickpea extract in the preparation of drugs and foods for prevention and treatment of obesity and NIDD.

The chickpea extracts studied in this invention include alcoholic extract and aqueous extract.
a. Preparation of alcoholic extract
   Pulverized chickpea powder (1-5 mm) is ultrasonically extracted for 4-5 times, each time over a period of 2 hours in a conical flask with 2-5 times its volume of ethanol. The extract solution was filtered with filter paper. The combined filtrates were concentrated under increased pressure at a temperature below 40°C to obtain the alcoholic extract.
b. Preparation of aqueous extract
   Pulverized chickpea powder (1-5 mm) is ultrasonically extracted for 3 times, each time over a period of 3 hours in a conical flask with 2-5 its volume of deionized water. The extracted solution was filtered and the filtrate was centrifuged. The supernatant was concentrated at the temperature below 60°C under reduced pressure to obtain the aqueous extract.
c. Preparation of chickpea extract
   Triturate the chickpea extract obtained from process a or b in a mortar with gradual addition of DMSO as suspension-forming agent. The suspension was enhanced further with ultrasonic pulverizer to disperse the solution evenly. The final preparation was a 10-times concentrated suspension mixture, in which the added DMSO concentration was 0.1%. The suspension mixture was diluted with distilled water before use to prepare a solution for drinking. The concentration of the extract in the drinking solution was 4-8 g/L.

Best mode of the invention:

This invention describes the application of chickpea extract in the preparation of drugs and foods for prevention and treatment of obesity and NIDD.

The chickpea (Cicer arietinum L.) used in this invention belongs to leguminous plants, including species of big and small particle size. The chickpea can be used immediately after being picked-up or be used after drying.

The polar solvents used in the extraction are water or organic polar solvents and their mixtures.

No special limits for the use of water are indicated. Any drinkable common water can be used, such as tap-water and well water; common water treated by distillation, ion exchange or ultra-filtration; or purified water after treatment by the combination of the above methods; natural water, such as underground water or spring water, etc.; water for injection: sterilized water or alkaline water, etc. Purified water is preferred among them and most preferred is deionized water purified by cation and anion exchange resins.

Organic polar solvents used in the methods of this invention include acetone, methylethylketone, acetonitrile, short chain alcohols, NMP, ethyl-ethylene glycol, DMSO, trichloroethylene, etc. Among C₁₋₄ alcohols, there are: methanol, ethanol, isopropanol, propanol, butyl alcohol, etc.

In the consideration of safety and as regard to the effects and further compatibility with diets, the preferred polar solvents used in chickpea extract are water, short chain alcohols, or DMSO.

Solvent mixtures denote the mixture of water and organic polar solvent, e.g.: alcoholic water solution of C₁₋₄ alcohol and water (such as: methanol water solution, ethanol water solution, isopropanol water solution, propanol water solution, butanol water solution, etc.). In the mixtures of water and organic polar solvent, the organic polar solvent concentration is about 0.1-99.5% (v/v), among them 30-99.5% (v/v) is preferred and 50-99.5% (v/v) is most preferred.

In this invention the polar solvent are water, C₁₋₄ alcohols and C₁₋₄ alcoholic water solution; C₁₋₄ alcohol and C₁₋₄ alcoholic water solution are preferred; and ethanol and ethanolic water solution are much more preferred. These also includes C₁₋₄ alcoholic water solution and water containing C₁₋₄ alcohol (contains less than 5% (v/v) of water) or water containing ethanol.

In the invention, the extraction method using chickpea as raw material is not a specially confined method. In the extraction, the chickpea can be used directly, or be processed after being pulverized, grounded or chopped. Extraction is best performed after pulverizing chickpea into 1-5 mm particles. Pulverization methods including application of mechanical pulverizer are commonly used in the methods of the invention.

The extraction methods adopted in the invention can be various generally used methods, and ultrasonic wave application can greatly improve extracting efficiency. Extraction can be carried out at ordinary or elevated temperatures. The temperature should be controlled at below the boiling point of polar solvents being used. Duration of extraction may be varied according to temperature conditions and extraction methods.

The filtration and separation methods can be those commonly used, e.g., filtration using filter paper, cloth (such as gauze, etc.), glass filter or glassine filter, centrifugation, squeezing, etc.

In the process of extraction, the extract residue commonly undergoes 1-10 times repeated extraction, more particularly undergoes 2-5 times repeated extraction with polar solutions. Repeated extraction and combination of the extracts can fully extract out components from the chickpea.

Polar solvents contained in the extract liquid can be removed by concentration under ordinary pressure, increased pressure, or reduced pressure. Concentration may be carried under ordinary or elevated temperature conditions, the latter may be preferred. The temperature should be controlled below the boiling point of the solvent, preferably at 30-70°C. The extract may be lyophilized after concentration by any of the above mentioned methods.

The preventive or therapeutic agents of this invention are compounded by active components of chickpea extract and can be used as medicine. The chickpea extract can be directly used as medicine. In general, it is much more recommended to use various dosage forms of medicine prepared from chickpea extract and pharmaceutical excipients, e.g.: capsules, tablets (including coated or multilayer tablets, such as sugar-coated tablets, enteric-coated tablets, etc.), oral solid preparations such as pulvis or granules, oral solutions, injections, and suppositories for internal use. These preparations can all be manufactured by routine methods.

Additives can be commonly used in solid form preparations such as capsules, tablets, pulvis, granules, etc., such as, for example, fillers (e.g. lactose, cane sugar, glucose, starch, crystalline cellulose etc.), binding agents (e.g. starch paste, hydroxypropyl-cellulose solution, carboxymethyl-cellulose, gum Arabic, gelatin solution, sodium alginate solution, etc.), disintegrant (such as starch, sodium carboxymethylcellulose, calcium carbonate, etc.), emollients (e.g., magnesium stearate, talcum powder, stearic acid, calcium stearate, etc.), surfactants (e.g., polysorbate 80, polyoxyethylenic-stiffened-castor oil, etc.), and thickeners (e.g., hydroxyethyl-cellulose, hydroxypropyl-cellulose, polyethylene alcohol, polyethylene glycol, etc.). Various coating materials (e.g. gelatin, can sugar, gum Arabic, cellulose acetate, copolymer of methylacrylic acid, m-phthalic acid hydroxypropyl-cellulose, carboxymethylethylcellulose, hydroxypropylmethyl cellulose, etc.) can be used for coating the surface of tablets or granules. Capsules can be hard capsules as well as microcapsules and soft capsules. Solid preparations can be produces by usual methods.

Oral liquid medicines are made from solvents or dispersives, e.g., chickpea extract are dissolveed with polar solvent diluent or dispersed into suspension. Such polar solvent diluents which are relatively harmless to human body include, e.g., water, ethanol, dimethylsulfoxide (DMSO), and ethanolic water solution; among these water, ethanolic water solution, or DMSO, and especially DMSO water solution, are preferred. The concentration of ethanolic water solution or DMSO water solution is usually about 0.01-50% (v/v); preferred concentration is about 0.01-20% (v/v), more preferred concentration is about 0.05-10% (v/v), and most preferred concentration is 0.1-1% (v/v).

The liquid preparations re produced in detail by following steps:
a) Pulverize the chickpea in a pulverizer (e.g., a motar); add a polar solvent diluent in the amount of 5-20 times by volume of the chickpea extract, e.g. a polar solvent diluent of about 0.05-0.2% (v/v) DMSO water solution. It is preferred to add the DMSO solution gradually in the amount of 8-12 times the volume quantity of chickpeas and triturate to produce a suspension solution;
b) Suspending further the suspension from step (a) ultrasonically and/or using a pulverizer to get an evenly dispersed micro-particulate suspension. The suspension contains about 5-20 times diluted chickpea extract; the preferred concentration is about 8-12 times diluted;
c) The micro-particulate suspension from step (b) is diluted with water (e.g., distilled water, purified water, etc.) before drinking. A dilution is made to produce a drinking solution containing 0.1-2% concentration of the extract, in which the preferred concentration is 0.2-1% and the most preferred concentration is 0.4-0.8%.

The liquid preparation is compatible with, e.g., saccharides (e.g.: cane sugar, sorbinose, fructose, etc.), glycols (e.g., polyethylene glycol, 1,2-propylene glycol, etc.), dispersives or thickeners (e.g.: gelatin, gum Arabic, hydroxyethyl-cellulose, hydroxypropyl-cellulose, polyethylene alcohol, polyethylene glycol, etc.), emulsifying agents (e.g. fatty acid glycerides, cane sugar fatty acid ester, etc.), solubilizing agents (gum Arabic, polysorbate 80, etc.), pH-adjusting agents (e.g., citric acid, tri-sodium citrate, etc.), and preservatives (p-hydroxybenzoates, sorbic acid, etc.)

Intravenous injections can be dispensed by isotonic water solution to prepare injections for intravenous injection or infustions. For example, injections can be prepared usually by choosing some aqueous solvents from distilled water, normal saline, glucose solution or glucose saline solution for injection and compounded with adjuvants to produce solutions, suspensions or dispersive solutions. Suppositories for enteric use can be prepared with matrixes, such as cocoa butter.

Another purpose of this invention is to provide chickpea extract-containing foods beneficial to combating obesity or NIDD. Foods of this invention may not only be chickpea extracts, but generally denote also foods produced from chickpea extract as raw material together with additives. These can be, for example, capsules, tablets (including coated tablets, such as sugar coated tablets, multilayer tablets or lozenges), solid constructed forms of powders or granules, and also liquid-constructed forms. As additives, there are, for example, excipients (lactose, dextrin, maize starch, crystalline cellulose, etc.), emolients (e.g. magnesium stearate, cane sugar fatty acid ester, fatty acid glycerides, etc.), disintegrating agents (calcium carboxymethyl cellulose, sodium carbonate, etc.), binding agents (starch paste, hydroxypropyl cellulose liquid, gum Arabic etc.), solubilizing agents (gum Arabic, polysorbate 80), sweeteners (cane sugar, fructose, glucose, honey, etc.), pigments (β-carotine, edible tar pigment, riboflavin, etc.), preservatives (sorbic acid, p-hydroxybenzoates, sulfites, etc.), thickener (sodium alginate, salt of carboxymethyl-cellulose polyethylacrylate), antioxidants (BHA, BHT, ascorbic acid, etc.), perfumes (peppermint, strawberry perfume, etc.), acidifying agents (citric acid, citrate, etc.), vitamins, mineral substances, amino acids, pigments, etc. Such solid preparations can be produced by usual methods. Liquid preparations can be produced by the same methods as described for oral liquids.

Foods and drinks of this invention can also be chickpea extract itself or ordinary foods and drinks to which solid or liquid constituted substances of chickpea extract had been added, e.g. snacks (chewing gum, candy, tarred sugar, chocolate, biscuit, small snacks, jelly, tablet foods, etc.), noodles (buckwheat noodle, etc.), dairy products (milk, ice cream, yoghurt, etc.), seasonings (thick broad bean sauce, soy sauce, etc), soup and beverages ( fruit juices, black tea, tea, carbonated beverages, sport beverages, etc.) and other common foods and health-care foods (nutritional beverages), etc.

Foods and drinks of this invention contain therapeutically-effective chickpea extract for treating and preventing obesity and NIDD. Accordingly, it is preferred to indicate on the food and drink packaging that these products have therapeutic effects to alleviate obesity and/or NIDD.

In addition, foods and drinks of this invention can be used as special purpose health-care foods for improving the symptoms of obesity and/or NIDD.

The percentage of chickpea extract contained to the above mentioned medicines or foods is about 10-95%, and 50-90% is preferable.

The preventive and therapeutic preparations in this invention can be used in prevent and treatment of obesity, especially those of internal organ fat accumulated type. Foods and drinks of this invention are also can be used in reducing and alleviating obesity, especially obesity of internal organs of the fat-accumulated type.

The preventive and therapeutic preparations of this invention can be used in the prevention and treatment of NIDD, especially insulin resistance in NIDD. Moreover, the foods and drinks in this invention can be used to suppress NIDD, especially insulin resistance in NIDD to prevent the occurrence the disease and improve the symptoms of diabetes. Furthermore, the preventive and therapeutic preparations of this invention are also useful for prevention and treatment of the complications of obesity and NIDD, such as hyperlipidemia, hypertension and arteriosclerosis (e.g., brain artery diseases, such as cerebral infarction; and coronary artery diseases, myocardial infarction), etc. Foods and drinks produced by methods of this invention are also effective for controlling and improving the above mentioned complications.

The quantities of chickpea extract to be added to foods or to be administrated can be changed according to the variation of obesity and symptoms of NIDD, individual needs and method of medication. For example, the adult daily oral dosage of chickpea extract can be 100-1000 mg, with 200-800 mg being preferred.

The chickpea extract, extracted by polar solvents (water or ethanol) is preferred to chickpea itself in the suppression of the increasing body weight during high fat feeding. That means the chickpea extract possesses the ability to prevent or suppression the obesity. Alcoholic extracts of chickpea are more active than water extracts and show better results than the effect produced by water extract even with concentration equals half of that of the chickpea water extract. The alcoholic extract also shows remarkable suppression effect to the adhesion of fat on the internal organs caused by high fat diet.

In addition, the chickpea extract can reduce the increasing of serum TC, cholesterol and LDL and suppress the reduction of HDL caused by high fat feeding, with results being much better than for unprocessed chickpeas. The extract, therefore, can particularly be used in prevention, improving and treatment of hyperlipidemia. Especially, the alcoholic extract of chickpea is more active than the water extract. As above, better results were obtained with alcoholic extracts as could be obtained with aqueous extracts, even if alcoholic extracts were used at only half the concentration of aqueous extracts. This means that alcoholic chickpea extracts are especially useful to prevent, improve and treat hyperlipidemia.

Moreover, chickpea extract can suppress blood sugar elevation, impaired glucose tolerance (IGT), and insulin resistance. It can also suppress the reduction of adponectin secretion and the increase of body fat content. These results indicate that the chickpea extract can be effective to NIDD, especially to NIDD with insulin resistance during suppression of body fat accumulation. The alcoholic extract exerts much more prominent effect. Therefore, the chickpea alcoholic extract can become an ideal medicine for the prevention and treatment of obesity and/or NIDD, especially the NIDD accompanied by insulin resistance.

### DESCRIPTION OF THE DRAWINGS

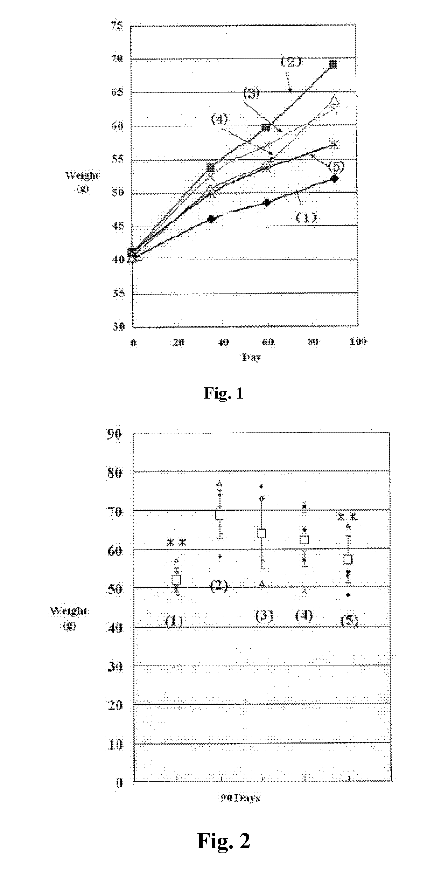

Fig. 1: Influence of chickpea extract (1) on body weight of high-fat diet mice

Chickpea alcoholic extract can lower the increase of body weight caused by high fat diet. Statistical significance appeared after 90 days of feeding **< 0.01. Mean + SD, 2Exp. (n=8-10/exp), *p<0.05,**<0.01: VS. high fat diet group 1) normal diet group(NFD), 2) high fat diet group(HFD), 3) high fat diet + chickpea diet group, 4) high fat diet + chickpea water extract group, 5) high fat diet + chickpea alcoholic extract group.

Fig. 2: Influence of chickpea extract on body weight of high-fat diet mice

The body weights after 90 days feeding with HFD, NFD, HFD + chickpea, HFD + aqueous extract, HFD + alcoholic extract were: 52.13±3.0 g, 69.00±6.26 g, 63.78±11.28 g, 62.33±7.04 g and 57.11±6.13 g, respectively. Body weight elevation was significantly reduced in the group of mice fed the high-fat diet plus alcoholic extract (p<0.01). Although a tendency toward reduction in the body weight were observed in chickpea group and aqueous extract group compared with HFD model group, but this difference did not yet reach statistical significance. (Mean Mean + SD, 2Exp. (n=8-10/exp), *p<0.05,**<0.01: vs. high fat diet group 1) normal diet group (NFD), 2) high fat diet group (HFD), 3) high fat diet + chickpea diet group, 4) high fat diet + chickpea water extract group, 5) high fat diet + chickpea alcoholic extract group.

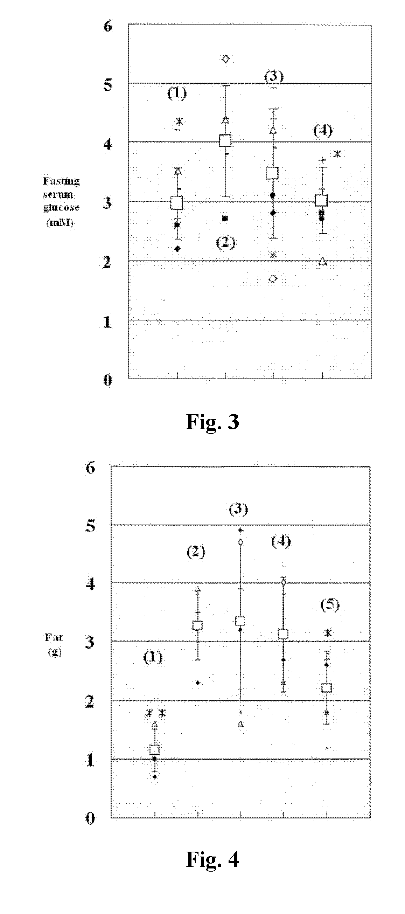

Fig. 3: Fasting serum glucose (FSG)
Divide mice into groups and measure the FSG after 60 days feeding. The FSG of NFD, HFD, HFD + chickpea, HFD + aqueous extract, HFD + alcoholic extract were 2.96 ± 0.6 mM, 4.01 ± 0.94 mM, 3.47 ± 1.1 mM and 3.01 ± 0.55 mM (Mean + SD, 2Exp. (n=8-10/exp), *p<0.05: VS. HFD) 1) NFD, 2) HFD, 3) HFD + aqueous extract, 4) alcohol HFD + alcoholic extract.

Fig. 4: Fat weight in internal organ (epididymis)
The body weight after 100 days feeding with NFD, HFD, HFD + chickpea, HFD + aqueous extract, HFD + alcoholic extract were: 1.15 ± 0.37 g, 3.28 ± 0.59 g, 3.35 ± 1.34 g, 3.12 ± 0.98 g and 2.22 ± 0.62 g, respectively, alcoholic extract group resulted significantly reduction of the increase fat weight in epididymis caused by high fat diet feeding (p<0.05), and no differences were found among chickpea group, aqueous extract group and HFD group. (Mean±SD,2Exp. (n=8-10/exp),* p < 0.05,: VS. HFD group) 1) NFD group, 2) HFD group, 3) HFD + chickpea group, 4) HFD + aqueous extract group, 5) HFD + alcoholic extract group.

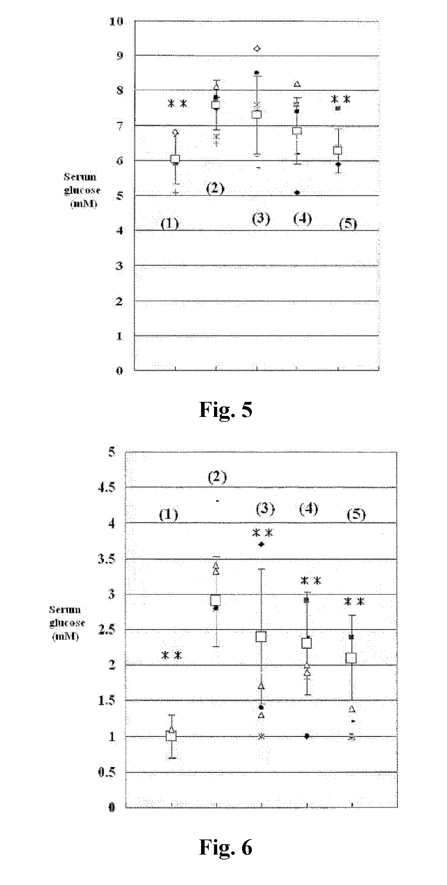

Fig. 5: Mice serum glucose levels at 2 hours after sugar loading
The alcoholic extract feeding mice group significantly decreases serum glucose level at 2 hrs after meal (<0.01), and no significant differences were found in the groups of simple chickpea feeding and water extract feeding(Mean±SD,2Exp. (n=8-10/exp),** < 0.01: vs. HFD group) 1) NFD group, 2) HFD group, 3) HFD + chickpea group, 4) HFD + aqueous extract group, 5) HFD+ ethanolic extract group.

Fig. 6: Serum glucose level at 180 min. after insulin tolerance test (ITT).
After 3 months feeding, in comparison with high fat diet group, all of the chickpea group, and the water extract and alcoholic extract group show remarkable decrease of blood sugar levels at 180 min after intraperitoneal injection of insulin into the mice (p<0.01). That means the reduction of insulin sensitivity caused by high fat diet had been improved to a great extent. (Mean±SD, 2Exp. (n=8-10/exp),* ** < 0.01: vs. HFD group) 1) NFD group, 2) HFD group, 3) HFD +chickpea group, 4) HFD + aqueous extract group, 5) HFD + alcoholic extract group.

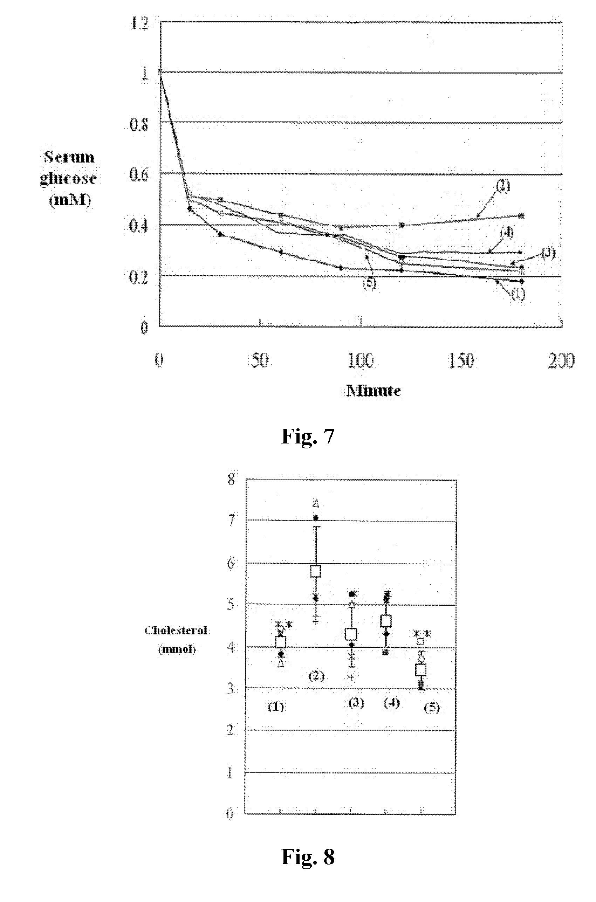

Fig. 7: The change of ITT blood sugar curve
All the chickpea, water extract and alcoholic extract groups have significantly enchanced the lowering of insulin sensitivity due to the long-term high fat diet feeding for 3 months all are p < 0.01.(Mean±SD,2Exp. (n=8-10/exp), ** < 0.01: VS. HFD group) 1)NFD group, 2) HFD group,3) HFD + chickpea group, 4) HFD + aqueous extract group, 5) HFD+ alcoholic extract group.

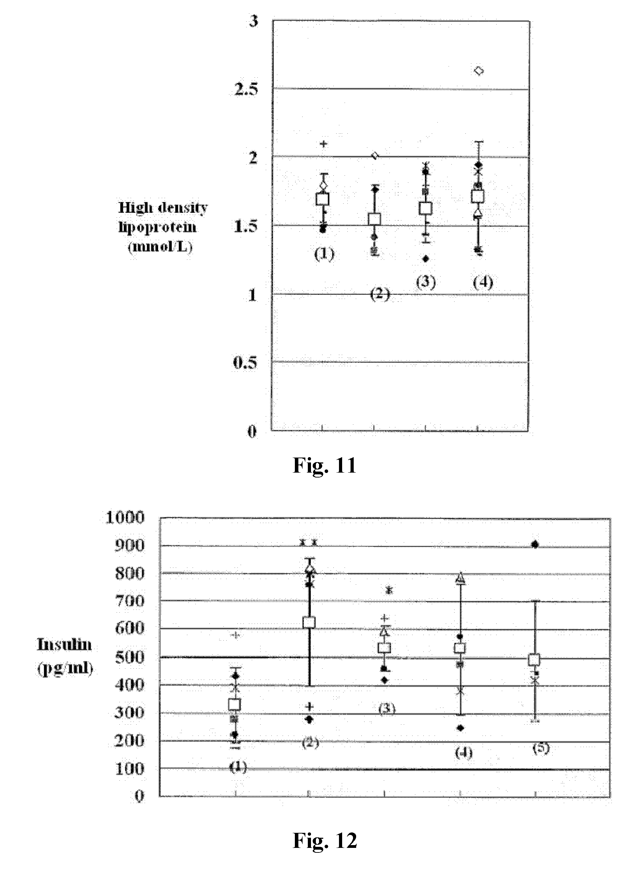

Figs. 8, 9, 10, 11: After 100 days feeding, all the chickpea groups and chickpea extract groups (large and small dosage) have significant improved by lowering of triglyceride, cholesterol and low density lipoprotein levels caused by high fat diet, and the therapeutic effect of alcoholic group is better than the other two groups. The alcoholic extract group even have the tendency to elevate high density lipoprotein, but this has no statistical significance (Mean±SD, 2Exp. (n=8-10/exp), *p<0.05, ** < 0.01: vs. HFD group) 1) NFD group, 2) HFD group, 3) HFD + chickpea group (except fig.11), 4) HFD + aqueous extract group, 5) HFD + alcoholic extract group.

Fig. 12: Compared with NFD group, insulin levels of HFD group, chickpea group and water extract groups are elevated 2 hrs after the meal, but the elevation in alcoholic extract group is not significant(Mean±SD,2Exp. (n=8-10/exp),* p < 0.05,** < 0.01 :VS. HFD group). 1) NFD group, 2) HFD group, 3) HFD +chickpea group, 4) HFD + aqueous extract group, 5) HFD + alcoholic extract group.

### DETAILED DESCRIPTION OF THE INVENTION

The inventor had tested the preventive and curative effects in obesity and IDD through the following experiments.

Example 1

Materials and methods:
1. Divide Kunming mice (about 40 g body weight) into 5 groups: normal diet group, high fat diet group, high fat diet + chickpea extrat group, high fat diet + chickpea alcoholic extract group (0.2 g/kg/day), high fat diet + chickpea water extract group (0.4 g/kg/day) and observe for 100 days.
2. Diet composition:
   (1) Common feeds: carbohydrate 60%, protein 22%, fat 10% and others 8%;
   (2) High fat feeds: prepared by mixing common feeds with lard, milk powder, fish powder and cane sugar. Among them carbohydrate 40%, protein 13%, fat 40% and others 7%;
   (3) High fat feeds plus chickpea: Replace 5 kg of common feeds from each 50 kg high fat feeds with 5 kg of chickpeas. The total quantity of heat and the proportions of carbohydrate, protein and fat are similar with high fat group.
3. Preparation of chickpea extract
   a. Preparation of alcoholic extract
      Add 2 kg of ethyl alcohol into pulverized 1-5 mm chickpea powder in a conical flask and extract with ultrasonic agitation 4-5 times, each time for 2 hrs. Filter with filter paper, and concentrate the combined filtrate under increased pressure at controlled temperature below 40°C to obtain alcoholic extract.
   b. Preparation of aqueous extract
      Place pulverized 1 kg of 1-5 mm chickpea powder into a beaker, add 2 kg deionized water and extract 3 times with ultrasonic agitation, each time 3 hrs. Filter with gauze and further centrifuge the filtrate. Concentrate the supernatant under reduced pressure at temperature below 60°C to obtain aqueous extract.
   c. Dispensing of chickpea extract:
      Triturate chickpea extract with gradual addition of DMSO as suspension-forming agent in a mortar and further treat the resulting product with ultrasonic pulverizer to disperse evenly into a 10 times concentrated suspension wherein the added DMSO concentration is 0.1%. Dilute the finished product with distilled water before use to prepare a drinking solution. The concentration of the chickpea extract in the drinking solution is 4-8 g/L.
4. Drinking manner of chickpea extract: Drink freely by mixing with drinkable water (0.4-0.8% v/v)
5. Insulin tolerance test (ITT): Intraperitoneally inject commonly used insulin with the dosage of 1 IU/kg, and determine the sugar level of the blood from the tail vein with freestyle glucometer at 0, 15, 30, 60, 90, 120, and 180 min.
6. Oral glucose tolerance test (OGTT) and insulin release test (IRT): For OGTT, use 20% glucose solution with dosage of 2 g/kg body weight for gastric administration, and draw blood from the tail vein at the time when the stomach is empty and 120 min. after sugar intake. Perform IRT simultaneously.
7. Behead mice after 100 days, separate out serum and determine serum triglyceride, cholesterol, HDL-C and LDL-C levels by biochemical methods. The determinations were performed at the Clinical Test Department in Shanghai Rei-jing Hospital.

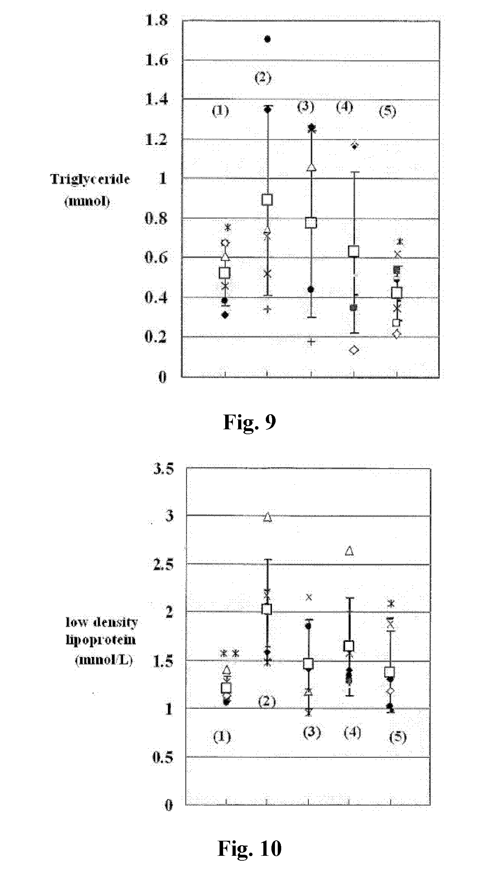

Results:
1. Influence of chickpea extract on body weight of high fat diet mice
After 100 days feeding, the body weights of NFD, HFD, HFD + chickpea, HFD + water extract and HFD + alcoholic extract were 52.13 ± 3.0 g, 69.00 ± 6.26 g, 63.78 ± 11/28 g, 62.33 ± 7.04 g, and 57.11 ± 6.13 g, respectively. This illustrates that the alcoholic extract can significantly reduce the body weight increase caused by high fat diet, and though the water extract group can also have the tendency of reducing the body weight, but without statistical significance (Fig. 1, 2).
2. Fasting blood sugar
Determine the fasting blood sugar of grouped mice on the 60th day of feeding. The blood sugar levels of NFD, HFD, water extract group and alcoholic extract group are 2.96 ± 0.6 mM, 4.01 ± 0.94 mM, 3.47 ± 1.1 mM and 3.01 ± 0.55 mM, respectively (Fig. 3).
3. Weight of internal organ fat (epididymis)
After 100 days feeding, the epididymial fat weights of the above five groups are 1.15 ± 0.37 g, 3.28 ± 0.59 g, 3.35 ± 1.34 g, 3.12 ± 0.98 g and 2.22 ± 0.62 g respectively. The alcoholic extract group can significantly reduce the increase of epididymial fat weights caused by high fat diet (p<0.05) but there are no differences of epididymial fat weights between the chickpea group, water extract group and high fat group (Fig. 4).
4. Influence of chickpea extract on the insulin sensitivity of long-term high fat feeding mice
After 90 days feeding, the lowering of insulin sensitivity due to the long-term high fat diet feeding in chickpea, water extract and alcoholic extract groups was significantly improved. The therapeutic effects to these three groups have no significant differences (Figs. 6, 7).
5. Influence of chickpea extract on the lipoprotein metabolism of long-term high fat feeding mice
After 100 days feeding, all the chickpea, water extract and alcoholic extract groups show significant improvement in the concentration of triglycerides, cholesterols and low density lipoprotein. Among them, the alcoholic extract group possessed superior therapeutic effect than other two groups (see following figure). The average high density lipoprotein levels in these three therapeutic groups are higher than the HFD group, but still have no statistical significance. (Figs. 9-11).

| | Triglycerides ( mmol/L ) | Cholesterols ( mmol/L ) | High density lipoprotein ( mmol/L ) ) |
|---|---|---|---|
| Normal control group | 0.52 ± 0.16* | 4.08 ± 0.34** | 1.20 ± 0.13** |
| High fat diet group | 0.89 ± 0.48 | 5.80 ± 1.06 | 2.02 ± 0.52 |
| HFD + chickpea group | 0.77 ± 0.47 | 4.28 ± 0.76* | 1.46 ± 0.46* |
| HDF + water extract group | 0.63 ± 0.40 | 4.60 ± 0.62* | 1.64 ± 0.51 |
| HDF + alcoholic extract group | 0.42 ± 0.14* | 3.44 ± 0.46**Δ | 1.38 ± 0.43* |

| | | | |
|---|---|---|---|
| Note: Compare with HFD group *p<0.05, **p<0.01; compare with HFD + chickpea groupΔp<0.05 | | | |

6. Mice blood sugar level and insulin level at 2 hrs after gastric administration of glucose
The chickpea alcoholic extract group reduced blood sugar level significantly at 2 hrs after meal but the chickpea group had no remarkable change; at the same time though the water extract group tends to reduce insulin level after meal, but still did not reach a statistical significance. In the mean time, in comparison with the NFD group, the HFD, chickpea diet and water extract groups elevated the insulin level at 2 hrs after meal, but the alcoholic extract groups did not show significant elevation (Figs. 5, 12).

| | Blood sugar (mmol/L) | Insulin (pg/ml) |
|---|---|---|
| Normal control group | 6.05 ± 0.71 ** | 327.22 ± 132.93 |
| HFD group | 7.59 ± 0.72 | 623.86 ± 230.73Δ |
| HFD + chickpea group | 7.30 ± 1.11 | 532.83 ± 82.88Δ |
| HFD + water extract group | 6.86 ± 0.95 | 529.63 ± 234.77Δ |
| HFD + alcoholic extract group | 6.29 ± 0.64** | 488.83 ± 214.54 |

| | | |
|---|---|---|
| Note: Compare with HFD group** p<0.01; compare with NFD groupΔp<0.01 | | |

Example 2

Materials and methods:
1. Divide Kunming mice ( about 40 g body weight) into 5 groups: normal diet group, high fat diet group, high fat diet + chickpea group, high fat diet + chickpea alcoholic extract group (0.2 g/kg/day), high fat diet + chickpea water extract group (0.4 g/kg/day) and performed observation for 100 days.
2. Diet composition:
   (1) Common feeds: carbohydrate 60%, protein 22%, fat 10% and others 8%;
   (2) High fat feeds: prepared by mixing common feeds with lard, milk powder, fish powder and cane sugar. Among them carbohydrate 40%, protein 13%, fat 40% and others 7%;
   (3) High fat feeds plus chickpea: Replace 5 kg common feeds from each 50 kg high fat feeds with 5 kg of chickpeas. The total quantity of heat and the proportions of carbohydrate, protein and fat are similar with high fat group.
3. Preparation of chickpea extract: Same as in Example 1.
4. Drinking of chickpea extract: Same as in Example 1.

Experiment 1: Observe body weight variation during the period of feeding
Weigh body weights of grouped mice at the days of 0, 30, 60 and 90. Observe the change of body weights during the period of feeding, and compare the significance of body weight differences within each group (see Fig. 1, 2). No difference of body weights were found at the beginning of experiment. After grouping and feeding for 30 days, difference of mice body weights within groups were appeared. Increasing of body weights in the mice of HFD group was obviously faster than other groups. Body weights in chickpea extract group, water extract group and normal control group were lower than that in high fat diet feeding group but the differences had no significance. After feeding for 90 days, the alcoholic extract group had better therapeutic effect than the water extract group and chickpea group, compared with high fat model group, p<0.05. This suggested that during the period of high fat diet feeding, the chickpea alcoholic extract treatment was able to avoid significant increase of mice body weight caused by high fat diet. The body weight was similar with normal control group when the quantity of intake was the same.

Experiment 2: Fasting and 2 hr after glucose loading blood sugar level of mice during the period of feeding
Determine the fasting and glucose loading blood sugar levels of grouped mice on the 60th day of feeding. Perform gastric administration of 50% glucose injection solution with a dosage of 2 g/kg body weight and determine the blood sugar after 2 hrs. Fasting diet is started at the night of one day before the day of experiment. Test begins 12 hrs later. In the test, draw blood from the tail vein at 0 and 2 hr and determine the blood sugar concentrations by a freestyle glucometer (see Figs. 3, 5). The chickpea significantly improved the impaired glucose tolerance caused by high fat feeding.

Experiment 3: Insulin tolerance test (ITT) by intraperitoneal injection of insulin
Insulin tolerance test: Intraperitoneally inject normal insulin with a dosage of 1 IU/kg and determine the blood sugar concentrations in the blood drawn from the tail vein with a freestyle glucometer at 0, 15, 30, 60, 90, 120 and 180 min. Use the blood sugar value determined at 0 min. as basal value to find the ratio between the blood values obtained at other times and the basal value and observe the lowering of blood sugar (see Figs. 6, 7). After 180 min., the blood sugar levels of chickpea diet group, water extract and alcoholic extract groups were all lower significantly than those of the model group. This suggests that the chickpea extract can correct the lowering of insulin sensitivity due to long-term high fat diet. The alcoholic extract group exhibited the most prominent effect.

Experiment 4: Determination of serum index
Determine fasting diet levels oftriglycerides (TG), total cholesterol (TC), high density lipoprotein (HDL-C) and low density lipoprotein (LDL-C) in grouped mice at 100 days feeding (see Figs. 8, 9, 10, and 11). Chickpea extracts can significantly decrease the elevation oftriglycerides, cholesterols and low density lipoprotein. The alcoholic extract group gave the best result and without prominent influences as to the level of high density lipoprotein.

Insulin level of mice at 2 hrs after glucose loading during the period of feeding
In comparison with the normal diet group, insulin levels at 2 hrs after glucose loading were elevated in the groups of high fat diet, chickpea diet and water extract, but the elevation was not significant in the group of alcoholic extract.

## Claims

1. A preparation containing chickpea extract for prevention or treatment of obesity and/or NIDD.

2. The preparation containing chickpea extract for prevention or treatment of obesity and/or NIDD of claim 1, **characterized** as a preventive or therapeutic preparation for obesity and/or NIDD caused by decrease secretion of adiponectin .

3. The preparation containing chickpea extract for prevention or treatment of obesity and/or NIDD of claim 1, **characterized** as a preventive or therapeutic preparation for obesity featured in the accumulation of fat in internal organ.

4. The preparation containing chickpea extract for prevention or treatment of obesity and/or NIDD of claim 1, **characterized** as a preventive or therapeutic preparation for NIDD, featured as the insulin resistant NIDD.

5. The preparation containing chickpea extract for prevention or treatment of obesity and/or NIDD of claim 1, **characterized in that** the described chickpea extract is obtained by the following steps:
(1) ground the chickpeas;
(2) extract the ground chickpeas with polar solvents; separate the extracted liquid and chickpea residue;
(3) extract the chickpea extracted residue obtained in step (2) with polar solvents; separate the extracted liquid and chickpea extracted residue again, and repeat the above steps;
(4) collect the extracted liquid and remove the polar solvents; and
(5) dry the obtained chickpea extract.

6. The preparation of claim 5, **characterized in that** the described solvent is a C1-4 alcohol or C1-4 alcoholic water solution.

7. The preparation of claim 6, **characterized in that** the described C1-4 alcohols are methyl alcohol, ethyl alcohol, isopropyl alcohol, propyl alcohol or butyl alcohol, among them ethyl alcohol or ethyl alcohol water solution being preferred.

8. The preparation of claim 6, **characterized in that** the concentrations of described polar solvents are 0.1-99.5% v/v, among them 30-99.5%v/v being preferred, and 50-99.5% v/v being most preferred.

9. A food containing chickpea extract possessing the effects of prevention or treatment of obesity and/or NIDD.

10. The food containing chickpea extract possessing the effects of prevention or treatment of obesity and/or NIDD of claim 9, **characterized in that** the food antagonizes the diseases caused by a decrease of adiponectin secretion.

11. The food containing chickpea extract possessing the effects of prevention or treatment of obesity and/or NIDD of claim 9, **characterized in that** the food antagonizes obesity caused by accumulation of fat in the internal organs.

12. The food containing chickpea extract possessing the effects of prevention or treatment of obesity and/or NIDD of claim 9, **characterized in that** the foods antagonize insulin resistance in NIDD patients.

13. The food containing chickpea extract possessing the effects of prevention or treatment of obesity and/or NIDD of claim 9, **characterized in that** the described chickpea extract is obtained by the following steps:
(1) ground the chickpeas;
(2) extract the ground chickpeas with polar solvents; separate the extracted liquid and chickpea residue;
(3) extract the chickpea extracted residue obtained in step (2) with polar solvents; separate the extracted liquid and chickpea extracted residue again, and repeat the above steps;
(4) collect the extracted liquid and remove the polar solvents; and
(5) dry the obtained chickpea extract.

14. The food of claim 13, **characterized in that** the described solvent is a C1-4 alcohol or a C1-4 alcoholic water solution.

15. The food of claim 14, **characterized in that** the described C1-4 alcohol is methyl alcohol, ethyl alcohol, isopropyl alcohol, propyl alcohol or butyl alcohol, among them ethyl alcohol or ethyl alcohol water solution being preferrable.

16. The food of claim 14, **characterized in that** the concentrations of described polar solvents are 0.1 -99.5% v/v, among them 30 -99.5%v/v being preferable, and 50 -99.5% v/v being most preferable.

17. The food containing chickpea extract possessing the effects of prevention or treatment of obesity and/or NIDD of claim 9, **characterized in that** the food is in the form of a tablet, a capsule, or a liquid drink.

18. The food containing chickpea extract possessing the effects of prevention or treatment of obesity and/or NIDD of claim 17, **characterized in that** the described liquid drinks are prepared from the following steps:
(1) extract the pulverized chickpea with alcohol; filter and separate the extract liquid and chickpea extract residue; extract again the separated chickpea extract residue with alcohol and filter; repeat the above process for many times and combine the filtrates; concentrate the combined filtrate under increased pressure at temperature below 40°C to obtain extract;
(2) pulverize the extract obtained from step (1) in a pulverizer, add 5-20 times their volume quantity of DMSO-water solution and further triturate to make a suspension; micronized the extract in the suspension liquid to disperse evenly in the DMSO-water solution; and
(3) dilute the suspension liquid from step (2) with water to form a liquid drink.

19. The food containing chickpea extract possessing the effects of prevention or treatment of obesity and/or NIDD of claim 17, **characterized in that** the described liquid drinks are prepared from the following steps:
(1) extract the pulverized chickpea with water; filter and separate the extract liquid and chickpea extract residue; centrifuge the filtrate to separate the supernatant and chickpea extract residue; repeat the above process for many times and combine the supernatants; concentrate the supernatant at below 60°C under reduced pressure to obtain the extract;
(2) pulverize the extract obtained from step (2) in a pulverizer, add 5-20 times quantity of DMSO-water solution and further disperse into a suspended liquid; micronized the suspended liquid to disperse evenly in the DMSO-water solution; and
(3) dilute the suspension liquid from step (2) with water to obtain a liquid drink.

20. The food containing chickpea extract possessing the effects of prevention or treatment of obesity and/or NIDD of claim 18 or 19, **characterized in that** in step (2) the addition quantity of DMSO-water solution is 5-20 times, preferred addition quantity of DMSO-water solution is 8-12 times, and the concentration is 0.05-0.2% v/v.

21. The food containing chickpea extract possessing the effects of prevention or treatment of obesity and/or NIDD of claim 18 or 19, **characterized in that** the liquid drink contains 0.1-2% chickpea extract, the better concentration is 0.2-1 % and the best is 0.4-0.8%.

22. The preparation containing chickpea extract for prevention or treatment of obesity and/or NIDD of claim 1, **characterized in that** the adult daily oral dosage is so that 100-1000 mg of chickpea extract are administered with 200-800 mg being preferred.

23. The food containing chickpea extract possessing the effects of prevention or treatment of obesity and/or NIDD of claim 9, **characterized in that** the adult daily oral dosage is so that 100-1000 mg of chickpea extract are administered with 200-800 mg being preferred.
